# EUROPEAN PATENT APPLICATION

(11) **EP 2 042 150 A1**
(43) Date of publication of application: **01.04.2009**
(21) Application number: 07790200.5
(22) Date of filing: 22.06.2007
(51) Int. Cl.: A61J 1/05, A61M 5/28, A61M 5/30

(54) **END PORTION FOR HERMETICALLY SEALED CONTAINER HAVING FINE OPENING SURFACE OBTAINED EASILY BY CLEAVAGE**

(30) Priority: 04.07.2006 JP 2006183957
(71) Applicant: Oyama, Yoshio, Tokyo 107-0052 (JP)
(72) Inventor: Oyama, Yoshio, Tokyo 107-0052 (JP)
(74) Representative: Pitchford, James Edward
(86) International application number: PCT/JP2007/000675
(87) International publication number: WO 2008/004337

(57) **Abstract**

An easy-to-open end portion of a hermetically sealed container whose opening surface is substantially flat and broken pieces are unlikely to be produced. The container comprises a tip portion (1), a truncated conical coupling portion (3) for coupling the tip portion (1) and a body portion (2), and a joint portion (4), wherein the tip portion (1), the joint portion (4), the coupling portion (3), and the body portion (2) are arranged coaxially to have a central axis (5), and space portions (14,13,12) are provided to extend continuously from the body portion (2) to the middle of the joint portion (4). When force is applied on the tip portion (1) from the lateral direction of the central axis (5), the joint portion (4) is broken, the head portion of the truncated conical portion (3) is exposed and the space portion (13) of the coupling portion becomes a free end.

## Description

### BACKGROUND OF THE INVENTION

### Field of the Invention

The present invention relates to an end portion of a hermetically sealed container having a fine opening surface obtained easily by cleavage. In particular, the present invention relates to an end portion of a hermetically sealed container which can be used as an ampoule usable for a needleless syringe.

### Description of the Related Art

In general, medical agents are orally administered. But, as for insulin administration for diabetic medication, interferon administration for treating hepatitis C virus infection or cancer, medical agents are injected using a syringe by a patient oneself. In these medical treatments, injection dosage needs to be continued. The routine injections have been a spiritual burden for patients, because of fear of the needles and pain thereof.

In recent years, however, needleless syringes have been developed and are used substituting for syringes having a needle. The medical administration using a needleless syringe is as follows. The needleless syringe shoots drug solution at high speed toward a skin, opening a small hole on the skin, and then the drug solution penetrates through the skin (see, for example, JP-A 2003-093508).

However, the above needleless syringe is difficult to handle, because it administers drug solution after having been put into vial and suctioned predetermined amount of drug solution. It is also not easy to suction proper amount of drug solution to be administered. Furthermore, there is a hygenic probleme that viruses attaches on the opening surface and propagates, bacause the same needleless syringe is repeatedly used for suction and administration. Thus, it might be better to prepare a hermetically sealed drug solution for each dosage and to administer it by opening it for each dosage.

On the other hand, there are hermetically sealed contianers for food and the like having an end portion, from which content such as soy sauce is let out by breaking the top portion by twisting the portion. This kind of hermetically sealed containers are widely used because of their convenience.

However, the hermetically sealed container is difficult to handle because the tip portion must be twisted to letting the contents out. In addition, since such containers are made from relatively soft plastic, when the top portion is broken by twisting, the opening surface will not be flat and burrs are left on the opening. On the other hand, when a hermetically sealed container is made from glass, broken pieces of glass are left by breaking the top portion. Therefore, the above mentioned edge portion of a hermetically sealed container is not suitable for a needleless syringe.

JP-A 09-266939 discloses an ampoule usable as a syringe. But this ampoule is not practical. In addition, the edge portion thereof is the same as that of conventional ampoule, and the opening surface cannot always be flat.

The object of the present invention is to provide an easy-to-open edge portion of a hermetically sealed container whose opening surface is substantially flat.

The object of the present invention is to provide an edge portion of a hermetically sealed container, in which broken pieces are unlikely to be produced even when a portion is opened by breaking the portion.

### SUMMARY OF THE INVENTION

The present invention is basically based on the following idea. An easy-to-open end portion of a hermetically sealed container can be provided, whose opening surface is substantially flat, by using an end portion of a hermetically sealed container which has a predetermined shape and is composed of a predetermined material. And since broken pieces are unlikely to be left on the end portion, the end portion can be used as a needless syringe.

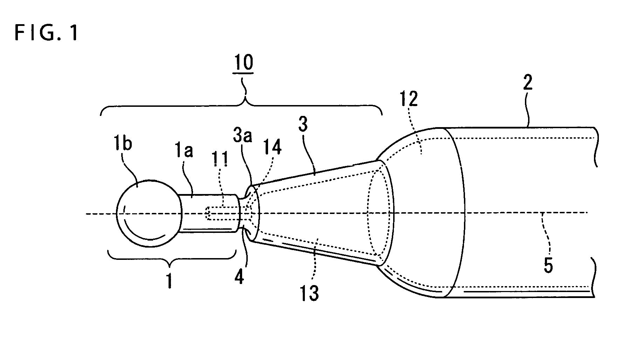

As shown in figure 1, the end portion of a hermetically sealed container according to the first aspect of the present invention basically comprises: a tip portion (1); a truncated conical coupling portion (3) for coupling the tip portion (1) and a body portion (2); and a joint portion (4) for coupling the tip portion (1) and the coupling portion (3), the joint portion (4) having narrower width than those of the tip portion (1) and the coupling portion (3), wherein the tip portion (1), the joint portion (4), the coupling portion (3), and the body portion (2) are arranged coaxially to have a central axis (5), wherein the tip portion (1), the joint portion (4), the coupling portion (3), and the body portion (2) have space portions (11, 14, 13, 12) being extended continuously from the body portion (2) to the middle of the tip portion (1), or space portions (14, 13, 12) being extended continuously from the body portion (2) to the middle of the joint portion (4), wherein the diameter of the space portion at the joint portion (4) ranges from 5 µm to 1 mm, and wherein the joint portion (4) is broken when a force is applied on the tip portion (1) from the lateral direction of the central axis (5), thereby exposing the head portion of the truncated conical coupling portion (3) and making the space portion (13) of the coupling portion a free end.

Since the coupling portion (3) is shaped in a truncated cone, and the width of the joint portion (4) is narrow, the joint portion (4) can easily be broken when a force is applied on the tip portion (1) from the lateral direction of the central axis (5), leaving the broken surface substantially flat. In this way, an easy-to-open end portion having fine and flat opening face can be easily and quickly obtained without twisting a top portion as in a prior art. The space portion (14) of the joint portion (4) and the space portion (11) of the tip portion (1) are preferred to have a cylindrical shape, a truncated conical shape, or a reverse truncated conical shape, each of which are arranged coaxially to have a central axis (5). The mean diameter thereof ranges, for example, from 5 µm to 1mm, and may range from 1x10⁻¹ mm to 1 mm. It is more preferred in the range from 1x10⁻¹ mm to 5x10⁻¹. Also, the diameter of the space portion (14) at the joint portion (4) connecting to the tip portion (1) may range, for example, from 0.15 mm to 0.17 mm. In particular, when the end portion of the hermetically sealed container of the present invention is used as an ampoule for a needleless syringe, for example, the diameter of the space portion is preferred to range from 5 µm to 1x10⁻¹ mm, and may be range from 1x10⁻² mm to 1x10⁻¹ mm. The penetration depth and the extent of diffusion can be controlled by varying the shape of the space portion or the mean diameter thereof.

As later described, since a free end is preferred to be obtained by breaking the joint portion of the end portion of a hermetically sealed container, the space portion (14, 13, 12) is preferred to be provided to extend continuously from the body portion (2) to the middle of the joint portion (4). But in terms of the productivity of the end portion of a hermetically sealed container, one that has the space portion (11, 14, 13, 12) extending continuously from the body portion (2) to the middle of the tip portion (1) is preferred. Fig.1 shows a columnar space extending continuously from the space portion (11) of the tip portion to the space portion (14) of the joint portion. But the columnar space may be tapered. The incline of the tapered portion (the conic apex angle which is made by extending the tapered portion) is, for example, from 1 degree to 45 degrees, it is preferred to be from 5 degrees to 30 degrees, and more preferably be from 10 degrees to 20 degrees, but it may also be from 5 degrees to 10 degrees. With these angles, the injection speed of drug solution can be accelerated by adjusting liquid current, thereby penetrating the agent into deeper layers of skin. On the other hand, the shape of the space portion may be widen toward the end. In this case, the above angles are reversed. This makes the injection speed of the drug solution slower, but the solution can be injected to a relatively wider area. Note that the shape of the space portion (13) of the coupling portion (3) is tapered toward the end, and the angle (apex angle) is preferred to be the same as that of the outer shape of the coupling portion (3). By so doing, the coupling portion (3) has uniformed strength. Also, the shape of the space portion (13) of the coupling portion may intentionally be formed such that it becomes different from the outer shape of the coupling portion. For example, it may have a portion being tapered toward the end. The incline of the portion is, for example, from 1 degree to 45 degrees, it is preferred to be from 5 degrees to 30 degrees, and more preferred to be from 10 degrees to 20 degrees, but it may also be from 5 degrees to 10 degrees.

A preferred embodiment of the end portion of a hermetically sealed container according to the first aspect of the present invention is the above described end portion of a hermetically sealed container, wherein the tip portion (1) comprises a columnar body portion (1a) and a head portion (1b), the columnar body portion (1a) being joined with the joint portion (4), the head portion (1b) being provided at the tip of the body portion, the head portion (1b) having a spherical shape or having a columnar shape, the diameter of the columnar shape being larger than that of the columnar body portion (1a).

As above mentioned, since the large head portion (1b) is provided on the tip portion (1), a large force can be added to the joint portion (4) by pressuring this head portion (1b) using "lever rule", thereby breaking the coupling portion easily. In addition, by exerting crosswise torque, fine and flat opening surface can be obtained quickly and easily. Note that if the axial length of the tip portion is too short, it is difficult to add force in the crosswise direction, and if the axial length is too long, it is inconvenient for carrying. So the length of the tip portion is, for example, from 3 mm to 5x10 mm, and more preferably from 5 mm to 1x10 mm. Note that the space portion (11), which is connected to the space portion (14) of the joint portion, is preferred to be provided to the middle of body portion (1b) of the tip portion. Due to the existence of this space portion (11), an opening portion can be obtained when the joint portion is broken.

Another preferred embodiment of the end portion of a hermetically sealed container according to the first aspect of the present invention is one of the above described end portion of a hermetically sealed container, wherein the average outside diameter of the joint portion (4) ranges from 1/4 to 1/2 (preferably 1/3 to 1/2) of the outside diameter of a head portion (3a) of the coupling portion (3).

Since the mean outside diameter of the joint portion (4) is in the above range, the joint portion can be easily broken when a force is added to the tip portion.

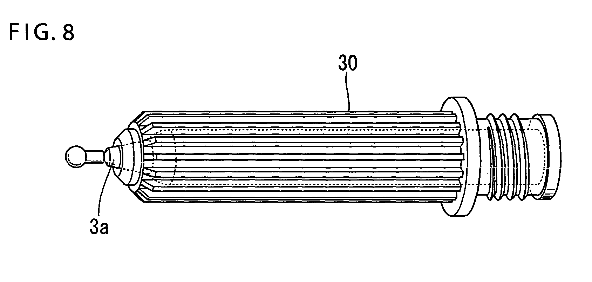

Another preferred embodiment of the end portion of a hermetically sealed container according to the first aspect of the present invention is one of the above described end portion of a hermetically sealed container, wherein a head portion (3a) of the coupling portion has a flat portion perpendicularly intersecting with the central axis (5). Note that the head portion (3a) is preferred to be shaped in tapered form (refer to Fig. 1 or Fig. 8). The height of the tapered portion is, for example, from 1x10⁻¹ mm to 5x10⁻¹ mm, and preferably be from 2x10⁻¹ mm to 4x10⁻¹ mm. The diameter of the head portion at the coupling portion is, for example, from 1 mm to 5x10 mm, and preferably be from 2 mm to 2x10 mm, and more preferably be from 3 mm to 1x10 mm. This achieves high adhesion between a skin and a needleless syringe comprising the end portion of a hermetically sealed container. Note that the tapered portion may be provided on the top of the joint portion. As shown in Fig. 8, the tapered portion may also be a portion that protrudes from the jacket (30) when the jacket (30) is put on the ampoule (20). The tapered portion means a portion which is shaped tapered toward a tip portion shown in, for example, a truncated cone.

Since the head portion (3a) of the coupling portion has a flat portion perpendicularly intersecting with the central axis (5), when the joint portion (4) is broken, it will be broken along this flat portion, thereby obtaining a broken surface which is nearly a flat surface and preventing generation of broken pieces. This flat portion is preferred to be arranged in concentric to the central axis (5).

Another preferred embodiment of the end portion of a hermetically sealed container according to the first aspect of the present invention is one of the above described end portion of a hermetically sealed container, wherein the length of the joint portion (4) in the central axis (5) direction ranges from 1x10⁻³ mm to 1 mm.

If a joint portion is too long, a broken surface will not be flat, and broken pieces are highly likely to be generated. On the other hand, if a joint portion is too short, it will be difficult to provide a joint portion. Thus the length of the joint portion (4) is, for example, from 1x10⁻³ mm to 1 mm, preferably from 1x10⁻³ mm to 1x10⁻² mm, and more preferably from 5x10⁻³ mm to 1x10⁻² mm.

Another preferred embodiment of the end portion of a hermetically sealed container according to the first aspect of the present invention is one of the above described end portion of a hermetically sealed container, wherein the tip portion (1), the joint portion (4), the coupling portion (3), and the body portion (2) are integrally formed, wherein the tip portion (1), the joint portion (4), the coupling portion (3), and the body portion (2) are composed of resin composition represented by the below-described general formula (I), and the number average molecular weight of the resin composition ranges from 1x10³ to 1x10⁶ (preferably 5x10³ to 5x10⁵, and more preferably 1x10⁴ to 1x10⁵).

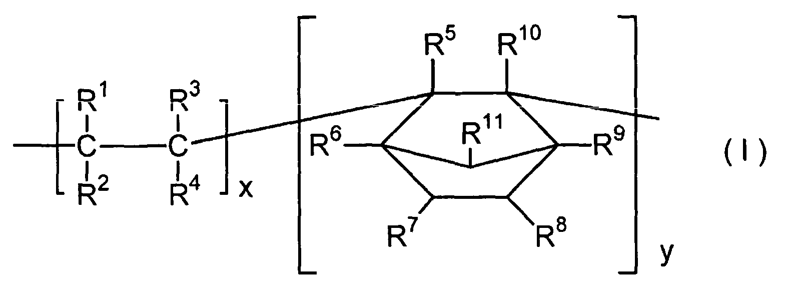

In the formula (I), R¹ to R⁴ are the same or different, and each represents a hydrogen atom, a C₁₋₃ alkyl group, a hydroxyl group, a methoxy group, an ethoxy group, a fluorine atom, a chlorine atom, or a bromine atom. Further, R⁵ to R¹¹ are the same or different, and each represents a hydrogen atom, a C₁₋₃ alkyl group (a methyl group, an ethyl groupe, or a plopyl group), a hydroxyl group, a methoxy group, an ethoxy group, a fluorine atom, a chlorine atom, or a bromine atom, or R⁷ and R⁸ together represents a C₄₋₆ bicyclo alkyl group, the C₄₋₆ bicyclo alkyl group has no substitution group or has one or more substitution groups selected from the group of "a methyl group, an ethyl group, a methoxy group, an ethoxy group, and a hydroxyl group". Preferred R⁵ to R¹¹ are the same or different, a hydrogen atom, a methyl group, a hydroxyl group, a methoxy group, an ethoxy group, a fluorine atom, or a chlorine atom, and more preferred R⁵ to R¹¹ are a hydrogen atom. The x and y show a ratio of substructure comprising ethylene based copolymer, and x:y is from 1:5 to 5:1 (preferably from 1:4 to 4:1, and more preferably from 1:3 to 3:1). Another preferred embodiment of the end portion of a hermetically sealed container according to the first aspect of the present invention is one of the above described end portion of a hermetically sealed container, wherein the glass translation temperature of the resin composition ranges from 140°C to 180°C.

Ampoules (20) produced from various materials were used for experiments of breaking a joint portion. When a material having too high crystalline was used in the experiment, the joint portion was broken and broken pieces were generated. On the other hand, when a material having sufficient malleability and ductility, such as PET, was used in the experiment, the joint portion was not successfully broken and burrs are left on the opening. In the present invention, since the ampoule is composed exclusively or mainly of the above resin composition, it has appropriate hardness. Therefore, when the joint portion is broken, a fine opening can be obtained, effectively preventing generation of broken pieces. And since the above resin composition is adopted as the material, the ampoule can easily be sterilized by soaking it in alcohol, or irradiating ultra violet light to it. As a result, it can easily be handled. Note that, since the unit molecule itself comprising the above resin composition is public knowledge, it can easily be obtained by obtaining unit molecules including a double bond respectively, mixing them with a predetermined ration, and combining them by addition polymerization. Also, the resin composition which is commercially available can be used.

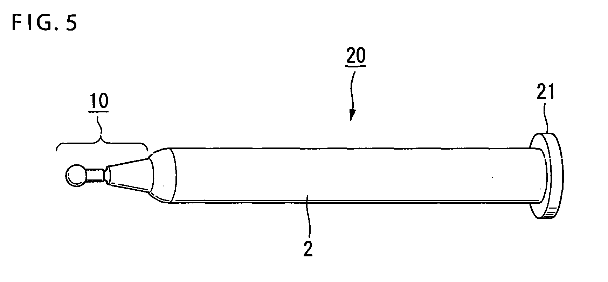

The second aspect of the present invention, as shown in Fig. 5, basically relates to an ampoule (20) comprising one of the above described end portions of a hermetically sealed container, and the body portion (2). And the third aspect of the present invention basically relates to an ampoule loading kit comprising the above described ampoule (20), the ampoule (20) according to claim 11; and a jacket (30) in which the ampoule (20) can be inserted, wherein a hole portion (31) is provided at the tip of the jacket (30), and wherein the tip portion (1), the joint portion (4) and a head portion (3a) of the coupling portion, or the tip portion (1) and the joint portion (4) protrude from the hole portion (31), the hole portion (31) being provided at the tip of the jacket, when the ampoule (20) is inserted in the jacket (30). In particular, for example, when an ampoule whose head portion (3a) of the coupling portion has tapered shape and protrudes from the jacket is used as a needleless syringe, high adhesion between the ampoule and a skin can be achieved. In addition, it is hygienically preferred, because the reusable jacket (30) can be prevented from touching the skin due to the existence of the tapered portion. From this perspective, the head portion (3a) is preferred to protrude from the jacket by 5x10⁻¹ mm to 5 mm, and more preferred to protrude by 1 mm to 3 mm.

Since a crosswise force is added to the head portion (3b) of an ampoule with a jacket put on, a force can be added to an ampoule which is in a fixed state. As a result, the joint portion (4) can easily be broken. The ampoule (20) is generally thin and is not easy to fix with fingers, but by putting a jacket (30) on it, the diameter of the ampoule is enlarged, which makes it easy to fix the ampoule with fingers. Also, as shown in the figure, multiple parallel protruding portions are provided around the jacket as finger stoppers. As a result, the jacket can be fixed with little force. Also, a connecting mechanism may be provided at the lower edge portion thereof in order to connect the jacket with the other apparatus.

The present invention can provide an easy-to-open end portion of a hermetically sealed container whose opening surface is substantially flat.

The present invention can further provide an easy-to-open end portion of a hermetically sealed container on which broken pieces are unlikely to be generated when a certain portion is broken to open the ampoule. As the result, the present invention can provide, for example, a preferred needleless syringe and the like.

### BRIEF DESCRIPTION OF THE DRAWINGS

Fig. 1 is a schematic diagram showing a basic structure of the end portion of a hermetically sealed container of the present invention.
Fig. 2 is a conceptual diagram showing a preferred embodiment of the end portion of a hermetically sealed container according to the first aspect of the present invention.
Fig. 3 is a conceptual diagram showing a preferred embodiment of the end portion of a hermetically sealed container according to the first aspect of the present invention.
Fig. 4 is a conceptual diagram showing a preferred embodiment of the end portion of a hermetically sealed container according to the first aspect of the present invention.
Fig. 5 is a schematic diagram showing a basic structure of the ampoule of the present invention.
Fig. 6 is a conceptual diagram showing a basic structure of the jacket of the present invention.
Fig. 7 is a conceptual diagram explaining the needleless syringe unit of the present invention.
Fig. 8 is a conceptual diagram showing an ampoule with the jacket put on.
Fig. 9 is a conceptual diagram showing an ampoule after use.
Fig. 10 is a photograph, replacing a diagram, showing a jacket and an ampoule comprising a needleless syringe unit.
Fig. 11 is a photograph, replacing a diagram, showing an ampoule on which a jacket is put and then the joint portion of the ampoule was broken.

### Element Numerals

- 1: tip portion
- 1a: columnar body portion
- 1b: head portion of a tip portion
- 2: body portion of a tip portion
- 3: coupling portion
- 3a: head portion of a coupling portion
- 4: joint portion
- 5: central axis
- 11-14: space portion

### DESCRIPTION OF THE PREFFERED EMBODIMENTS

Hereinafter, the end portion of a hermetically sealed container of the present invention is explained according to the figures. Fig. 1 is a schematic view showing a basic structure of the end portion of a hermetically sealed container of the present invention. As shown in Fig. 1, the end portion of a hermetically sealed container of the present invention comprises: a tip portion (1); a truncated conical coupling portion (3) for coupling the tip portion (1) and a body portion (2); and a joint portion (4) for coupling the tip portion (1) and the coupling portion (3), the joint portion (4) having narrower width than those of the tip portion (1) and the coupling portion (3), wherein the tip portion (1), the joint portion (4), the coupling portion (3), and the body portion (2) are arranged coaxially to have a central axis (5), wherein the tip portion (1), the joint portion (4), the coupling portion (3), and the body portion (2) have space portions (11, 14, 13, 12) being extended continuously from the body portion (2) to the middle of the tip portion (1), or space portions (14, 13, 12) being extended continuously from the body portion (2) to the middle of the joint portion (4).

Since the end portion of a hermetically sealed container of the present invention comprises the above structure, the joint portion (4) is broken when a force is applied on the tip portion (1) from the lateral direction of the central axis (5), thereby exposing the head portion of the truncated conical coupling portion (3), and the space portion (13) of the coupling portion becomes a free end.

Since the coupling portion (3) is shaped in a truncated cone, and the width of the joint portion (4) is narrow, the joint portion is easily broken when a force is applied on the tip portion (1) from the lateral direction of the central axis, leaving the broken surface substantially flat. In this way, an easy-to-open end portion having fine and flat opening face can be easily and quickly obtained without twisting a top portion as in a prior art. The space portion (14) of the joint portion (4) and the space portion (11) of the tip portion (1) are preferred to have a cylindrical shape, a truncated conical shape, or a reverse truncated conical shape arranged coaxially to have a central axis (5). The mean diameter thereof ranges, for example, from 1x10⁻¹ mm to 1 mm. It is more preferred in the range from 1x10⁻¹ mm to 5 x10⁻¹. The penetration depth and the extent of diffusion can be controlled by varying the shape of the space portion or the mean diameter.

As later described, since a free end is obtained by breaking the joint portion of the end portion of a hermetically sealed container, the space portion (14, 13, 12) is preferred to be extended continuously from the body portion (2) to the middle of the joint portion (4). But in terms of the productivity of the end portion of a hermetically sealed container, one that has the space portion (11, 14, 13, 12) extending continuously from the body portion (2) to the middle of the tip portion (1) is preferred. Fig.1 shows a columnar space extending continuously from the space portion (11) of the tip portion to the space portion (14) of the joint portion. But the columnar space may be tapered. The inclination of the tapered portion (the conic apex angle which is made by extending the tapered portion) is, for example, from 1 degree to 45 degrees, it is preferred to be from 5 degrees to 30 degrees, and more preferably from 10 degrees to 20 degrees, but it may also be from 5 degrees to 10 degrees. With these angles, the injection speed of drug solution can be accelerated by adjusting liquid current, thereby penetrating the agent into deeper layers of skin. On the other hand, the shape of the space portion may be widen toward the end. In this case, the above angles are reversed. This makes the injection speed of the drug solution slower, but the solution can be injected to a relatively wider area. Note that the shape of the space portion (13) of the coupling portion (3) is tapered toward the end, and the angle (apex angle) is preferred to be the same as that of the outer shape of the coupling portion (3). By so doing, the coupling portion (3) can be made to have uniformed strength. Also, the shape of the space portion (13) of the coupling portion may intentionally be formed such that it becomes different from the outer shape of the coupling portion. For example, it may have a portion being tapered toward the end. The inclination of the portion is, for example, from 1 degree to 45 degrees, it is preferred to be from 5 degrees to 30 degrees, and more preferably from 10 degrees to 20 degrees. It is desirable that loss of current be small to send drug solution from an ampoule into living body with high pressure. And so it is preferred that separation phenomenon of current be prevented as much as possible. From hydrodynamic view point, it is considered that the loss can be lowered when the apex angle (aperture) is set to be 15 degrees. On the other hand, if the drug solution is injected in a wider area, it is highly likely that the drug solution penetrate through pain spots in the skin, thereby causing a pain by the injection. From this perspective, the apex angle is set to be, for example, from 1 degree to 10 degrees, preferably from 5 degrees to 10 degrees. Also, the space portion (14) provided in the joint portion (4) or the portion connected to the space portion (14) between the space portion (14) and the space portion (13) of the coupling portion (3) may have a columnar portion. In this case, when the height (the length) of the space of the columnar portion is represented by L, and the sectional diameter is represented by D, if L/D is above 5, separation phenomenon occurs. When L/D is small, loss of the current is small. But the pressure exerted on the tip of an ampoule is high, and so there is a possibility of breaking the ampoule. From this perspective, L/D is preferably from 0.1 to5, may be from 0.5 to 3, or from 0.5 to 2, may also be from 1 to 2. When L/D is 2 or below, extremely strong pressure is exerted at the tip portion of the ampoule. So, the maximum thickness of the coupling portion (3) is preferred to be from 2.5 mm to 1 cm, and is more preferred to be from 2.5 mm to 5 mm.

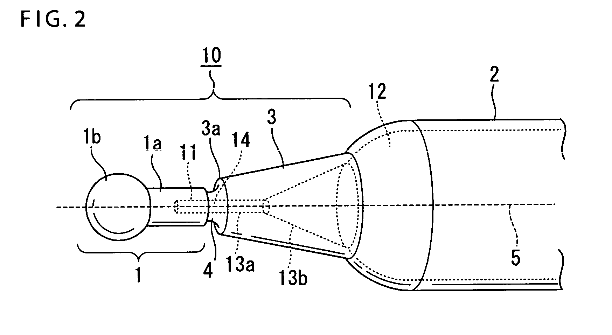

Fig. 2 is a conceptual diagram showing a preferred embodiment of an end portion of a hermetically sealed container according to the first aspect of the present invention. As shown in Fig. 2, a space portion (13) of the coupling portion (3), for example, has a columnar portion (13a) which is connected to the space portion (11) of the tip portion (1) and a truncated cone shaped portion (13b) whose bottom is connected to the space portion (12) of the body portion (2). In this way, by intentionally providing a columnar portion, the current of drug solution can be controlled, thereby adjusting the penetration depth or the spread area of the drug solution. Note that the columnar portion (13a) of the coupling portion (3) may be shaped in a truncated cone which has low apex angle compared to the truncated cone shaped portion (13b). As shown in Fig. 2, for example, the height (length) of the columnar portion (13a) is equal to or lower (shorter) than the height (length) of the truncated cone shaped portion (13b). The height of the columnar portion (13a) is, for example, from 10% to 90% of the height of truncated cone shaped portion (13b). The ratio may be from 20% to 80%, and it may also he from 30 % to 70%.In this way, by adjusting the height of the columnar portion (13a) and that of the truncated cone shaped portion (13b) as appropriate, the current of the drug solution can be controlled. When the height (the length) of the columnar portion is represented by L, and the sectional diameter is represented by D, if L/D is above 5, separation phenomenon occurs. When L/D is small, loss of the current is small. But the pressure exerted on the tip of an ampoule becomes high, and so there is a possibility of breaking the ampoule. From this perspective, L/D is preferably from 0.1 to 5, may be from 0.5 to 3, or from 0.5 to 2, may also be from 1 to 2. When L/D is 2 or below, extremely strong pressure is exerted at the tip portion of the ampoule. So, the maximum thickness of the coupling portion (3) is preferred to be from 2.5 mm to 1 cm, and is more preferred to be from 2.5 mm to 5 mm.

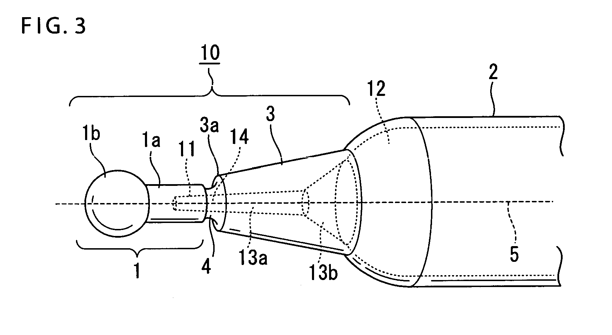

Fig. 3 is a conceptual diagram showing a preferred embodiment of an end portion of a hermetically sealed container according to the first aspect of the present invention. As shown in Fig. 3, a space portion (13) of the coupling portion (3), for example, has a columnar portion (13a) connected to the space portion (11) of the tip portion (1), and a truncated cone shaped portion (13b) whose bottom is connected to the space portion (12) of the body portion (2). The one shown in Fig.3 has a height (length) of the columnar portion (13a) which is higher than that of the truncated cone shaped portion (13b). The height of the truncated cone shaped portion (13b) is, for example, from 10% to 90% of the height of the columnar portion (13a). The ratio may be from 20% to 80%, and it may also be from 30 % to 70%. In this way, by adjusting the height of the columnar portion (13a) and that of the truncated cone shaped portion (13b) as appropriate, the current of the drug solution can be controlled. Note that the columnar portion (13a) of the coupling portion (3) may have a columnar shape having a smaller apex angle compared to that of the truncated cone shaped portion (13b). The shape of the columnar portion (13a) is preferred to be shaped in a truncated cone as shown in Fig. 3. It is desirable that loss of current be small to send drug solution from an ampoule into living body with high pressure. And so it is preferred that separation phenomenon of current be prevented as much as possible. From hydrodynamic view point, it is considered that the loss can be lowered when the apex angle (aperture) is set to be about 15 degrees. On the other hand, if the drug solution is injected in a wider area, it is highly likely that the drug solution penetrate through pain spots in the skin, thereby causing a pain by the injection. From this perspective, the apex angle is set to be, for example, from 1 degree to 10 degrees, preferably from 5 degrees to 10 degrees.

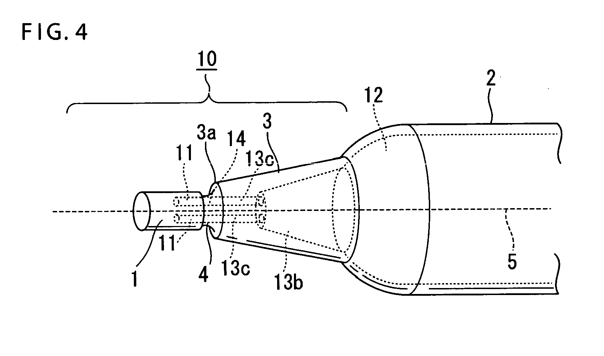

Fig. 4 is a conceptual diagram showing a preferred embodiment of the end portion of a hermetically sealed container according to the first aspect of the present invention. As shown in Fig. 4, the space portion (13) of the coupling portion (3) may include multiple cylindrical space portions (13c) which are independently provided in parallel with each other. In particular, 2 or 3 columns are preferred to be arranged symmetrically to the central axis (5). With this configuration, liquid drugs can be dispersedly administered, thereby minimizing the damage to a skin. Also, the space portions may be a combination of a columnar or a truncated conical (either tapered or widen toward the end) space portion, which are provided around a central axis, and 2 to 4 columnar or truncated conical space portions which are arranged symmetrically to the above space portion. In particular, it is preferred that the volume of the central space portion and that of the space portion arranged around it be different. The specific volume of the central space portion is preferred to be from 1.1 to 2 times of the volume of the space portions around the central space portion. With these configurations, liquid drugs having various flow rates can be obtained, thereby being able to administer them into deep layers or shallow layers of a skin. When the height (the length) of the 2 to 4 columnar space portions is represented by L, and the sectional diameter is represented by D, if L/D is above 5, separation phenomenon occurs. When L/D is small, loss of the current is small. But the pressure exerted on the tip of an ampoule becomes high, and so there is a possibility of breaking the ampoule. From this perspective, L/D is preferably from 0.1 to 5, may be from 0.5 to 3, or from 0.5 to 2, may also be from 1 to 2. When L/D is 2 or below, extremely strong pressure is exerted at the tip portion of the ampoule. So, the maximum thickness of the coupling portion (3) is preferred to be from 2.5 mm to 1 cm, and is more preferred to be from 2.5 mm to 5 mm.

A preferred embodiment of the end portion of a hermetically sealed container according to the first aspect of the present invention is one of the above described end portion of a hermetically sealed container, wherein the tip portion (1) comprises a columnar body portion (1a) and a head portion (1b), the columnar body portion (1a) being joined with the joint portion (4), the head portion (1b) being provided at the tip of the body portion, the head portion (1b) having a spherical shape or having a columnar shape, the diameter of the columnar shape being larger than that of the columnar body portion (1a).

As above mentioned, since the large head portion (1b) is provided on the tip portion (1), a large force can be added to the joint portion (4) by pressuring this head portion (1b) using "lever rule", thereby breaking the joint portion easily. In addition, by exerting crosswise torque, fine and flat opening surface can be obtained quickly and easily. Note that if the axial length of the tip portion is too short, it is difficult to add force in the crosswise direction, and if the axial length is too long, it is inconvenient for carrying. So the length of the tip portion is, for example, from 3 mm to 5x10 mm, and more preferably from 5 mm to 1x10 mm. Note that the space portion (11), which is connected to the space portion (14) of the joint portion, is preferred to be provided to the middle of the body portion (1b) of the tip portion. Due to the existence of this space portion (11), an opening portion can be obtained when the joint portion is broken.

Another preferred embodiment of the end portion of a hermetically sealed container according to the first aspect of the present invention is one of the above described end portion of a hermetically sealed container, wherein the average outside diameter of the joint portion (4) ranges from 1/4 to 1/2 (preferably 1/3 to 1/2)of the outside diameter of a head portion (3a) of the coupling portion (3).

Since the mean outside diameter of the joint portion (4) is in the above range, the joint portion can be easily broken when a force is added to the tip portion.

Another preferred embodiment of the end portion of a hermetically sealed container according to the first aspect of the present invention is one of the above described end portion of a hermetically sealed container, wherein a head portion (3a) of the coupling portion has a flat portion perpendicularly intersecting with the central axis (5). Note that the head portion (3a) is preferred to be shaped in tapered form (refer to Fig. 1 or Fig. 8). The height of the tapered portion is, for example, from 1x10⁻¹ mm to 5x10⁻¹ mm, and preferably from 2x10⁻¹ mm to 4x10⁻¹ mm. The diameter of the head portion at the coupling portion is, for example, from 1 mm to 5x10 mm, and preferably from 2 mm to 2x10 mm, and more preferably from 3 mm to 1x10 mm. This achieves high adhesion between a skin and a needleless syringe comprising the end portion of a hermetically sealed container. Note that the tapered portion may be provided on the top of the joint portion. As shown in Fig. 8, the tapered portion may also be a portion that protrudes from the jacket (30) when the jacket (30) is put on the ampoule (20). The tapered portion means a portion which is shaped tapered toward a tip portion shown in, for example, a truncated cone.

Since the head portion (3a) of the coupling portion has a flat portion perpendicularly intersecting with the central axis (5), when the joint portion (4) is broken, it will be broken along this flat portion, thereby obtaining a broken surface which is nearly a flat surface and preventing generation of broken pieces. This flat portion is preferred to be arranged in concentric to the central axis (5).

Another preferred embodiment of the end portion of a hermetically sealed container according to the first aspect of the present invention is one of the above described end portion of a hermetically sealed container, wherein the length of the joint portion (4) in the central axis (5) direction ranges from 1x10⁻³ mm to 1 mm.

If a joint portion is too long, a broken surface will not be flat, and broken pieces are highly likely to be generated. On the other hand, if a joint portion is too short, it will be difficult to provide a joint portion. So the length of the joint portion (4) is, for example, from 1x10⁻³ mm to 1 mm, preferably from 1x10⁻³ mm to 1x10⁻² mm, and more preferably from 5x10⁻³ mm to 1x10⁻² mm.

Another preferred embodiment of the end portion of a hermetically sealed container according to the first aspect of the present invention is one of the above described end portion of a hermetically sealed container, wherein the tip portion (1), the joint portion (4), the coupling portion (3), and the body portion (2) are integrally formed, and wherein the tip portion (1), the joint portion (4), the coupling portion (3), and the body portion (2) are composed of resin composition represented by the below-described general formula (I), and the number average molecular weight of the resin composition ranges from 1x10³ to 1x10⁶(preferably 5x10³ to 5x10⁵, and more preferably 1x10⁴ to 1x10⁵).

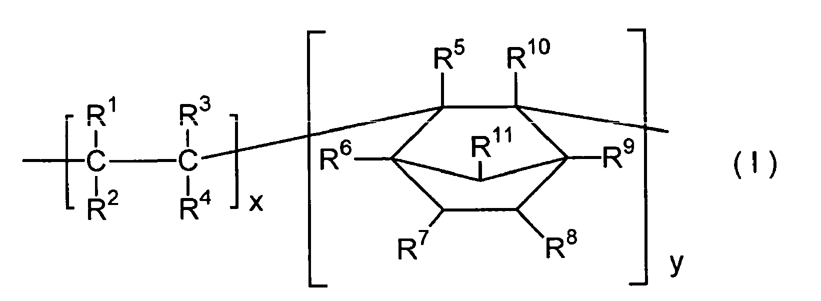

In the formula (I), R¹ to R⁴ are the same or different, and each represents a hydrogen atom, a C₁₋₃ alkyl group, a hydroxyl group, a methoxy group, an ethoxy group, a fluorine atom, a chlorine atom, or a bromine atom. Further, R⁵ to R¹¹ are the same or different, and each represents a hydrogen atom, a C₁₋₃ alkyl group (a methyl group, an ethyl groupe, or a plopyl group), a hydroxyl group, a methoxy group, an ethoxy group, a fluorine atom, a chlorine atom, or a bromine atom, or R⁷ and R⁸ together represents a C₄₋₆ bicyclo alkyl group, the C₄₋₆ bicyclo alkyl group has no substitution group or has one or more substitution groups selected from the group of "a methyl group, an ethyl group, a methoxy group, an ethoxy group, and a hydroxyl group". Preferred R⁵ to R¹¹ are the same or different, a hydrogen atom, a methyl group, a hydroxyl group, a methoxy group, an ethoxy group, a fluorine atom, or a chlorine atom, and more preferred R⁵ to R¹¹ are a hydrogen atom. The x and y show a ratio of substructure comprising ethylene based copolymer, and x:y is from 1:5 to 5:1 (preferably from 1:4 to 4:1, and more preferably from 1:3 to 3:1). Another preferred embodiment of the end portion of a hermetically sealed container according to the first aspect of the present invention is one of the above described end portion of a hermetically sealed container, wherein the glass translation temperature of the resin composition ranges from 140°C to 180°C.

Ampoules (20) produced from various materials were used for experiments of breaking a joint portion. When a material having too high crystalline was used in the experiment, the joint portion was broken and broken pieces were generated. On the other hand, when a material having sufficient malleability and ductility, such as PET, was used in the experiment, the joint portion was not successfully broken and burrs are left on the opening. In the present invention, since the ampoule is composed exclusively or mainly of the above resin composition, it has appropriate hardness. Therefore, when the joint portion is broken, a fine opening can be obtained, effectively preventing generation of broken pieces. Also, since the above resin composition was adopted as the material, the ampoule can easily be sterilized by soaking it in alcohol, or irradiating ultra violet light to it. As a result, it can easily be handled. Note that, since the unit molecule itself comprising the above resin composition is public knowledge, it can easily be produced by obtaining unit molecules including a double bond respectively, mixing them with a predetermined ratio, and combining them by addition polymerization. Also, the resin composition which is commercially available can be used.

### An ampoule usable for a needleless syringe

An example in which the end portion of a hermetically sealed container of the present invention is used as a needleless syringe is explained. Fig. 5 is a schematic diagram showing a basic structure of the ampoule of the present invention. As shown in Fig. 5, the ampoule usable for a needleless syringe is an ampoule comprising an ampoule body (2), and an end portion of a hermetically sealed container (10). A movable stopper having a diameter substantially the same as the inner diameter of the ampoule body is preferred to be provided inside the ampoule body (2). And drug solution is preferred to be hermetically sealed in between the movable stopper and a sealing portion (sealing edge of the tip portion). Note that, in Fig. 5, ampoule end portion (21) is provided at the end portion of the ampoule, and the diameter thereof is larger than that of the ampoule body (2) (e.g. 1.1 to 2 times of the diameter of the ampoule body, preferably 1.2 to 1.5 times thereof). With this end portion, the jacket (30) can be put on the ampoule (20) by pushing the ampoule end portion with fingers easily when the jacket (30) is put on the ampoule (20).

The ampoule body (2) is preferred to have a cylindrical smooth inner wall so that the movable stopper can slide. Note that the amount of drug solution accommodated in the ampoule is, for example, from 1x10⁻¹ ml to 1x10 ml, and may be from 1x10⁻¹ ml to 1 ml. Furthermore, when a very small amount of drug solution is administered in case of gene therapy, it may be from 1x10⁻³ ml to 1x10⁻¹ ml. The ampoule body (2) is preferred to be provided with an impact absorbing portion, which absorbs impact shocks and noises, at the portion adjacent to the end portion of a hermetically sealed container. Note that, in order to move the movable stopper, the movable stopper is preferred to be coupled with a plunger. The movable stopper is, for example, one or two flexible balls, and the diameter thereof is the same as the inside diameter of the ampoule, or it may be from 1.01 to 1.1 times of the inside diameter of the ampoule. The movable stopper is preferred to be shaped in streamlined tapering toward the tip of the ampoule.

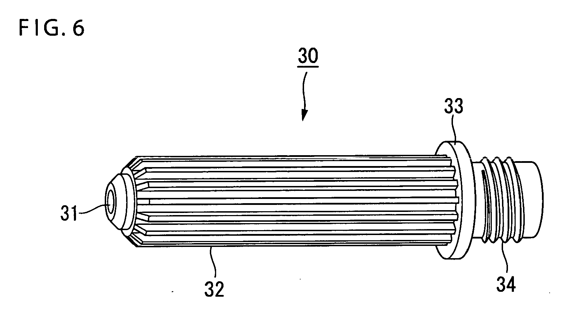

The needless syringe unit of the present invention comprises an ampoule and a jacket. Fig. 6 is a conceptual diagram showing a basic structure of the jacket of the present invention. As shown in Fig. 6, the jacket of the present invention basically is a jacket (30) in which the ampoule (20) can be inserted, wherein a hole portion (31) is provided at the tip of the jacket (30), and wherein the tip portion (1), the joint portion (4) and a head portion (3a) of the coupling portion, or the tip portion (1) and the joint portion (4) protrude from the hole portion (31), the hole portion (31) being provided at the tip of the jacket, when the ampoule (20) is inserted in the jacket (30). In Fig. 6, the reference number 32 identifies convex/concave portions which are provided around the jacket and extending toward the axis (5) direction. The reference number 33 identifies a skirt portion of the jacket whose circumference is made to be larger than those of the other portions. The body portion of a needleless syringe (a portion having pressuring mechanism and the like) is attached at the lower side of the skirt portion. The reference number 34 identifies a screw portion for connecting the ampoule with the needleless syringe body. The ampoule (20) can easily be connected to a needleless syringe body with this screw portion by turning the jacket (30) in which the ampoule is loaded.

### Production Method

The end portion of a hermetically sealed container, the ampoule, and the jacket of the present invention is preferred to be produced by a known method of producing an ampoule and the like. Hereinafter, the end portion of a hermetically sealed container of the present invention is preferred to be produced as an ampoule which comprises an end portion of a hermetically sealed container. Although a molding method includes the injection molding (including the powder injection molding method), the extrusion molding, and the like, the injection molding using a predetermined mold can preferably be used. In this case, materials above mentioned can be used as appropriate.

### Usage Method

Next, the method of using the end portion of a hermetically sealed container is explained. Basically, the joint portion (4) is broken when a force is applied on the tip portion (1) from the lateral direction of the central axis (5), thereby exposing the head portion of the truncated conical coupling portion (3), and the space portion (13) of the coupling portion becomes a free end. Applying this function, for example, the end Portion of a hermetically sealed container of the present invention can be used as an ampoule of a needleless syringe. Hereinafter, the usage method of the above embodiment is explained.

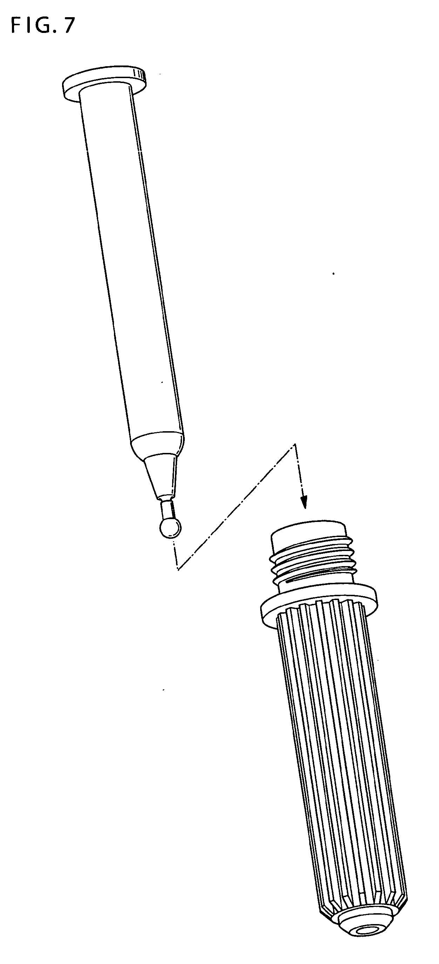

Fig. 7 is a conceptual diagram explaining a method for using the needleless syringe unit of the present invention (a method for loading the jacket (30) (a holder 30) with the ampoule (20)). Fig. 8 is a conceptual diagram showing an ampoule with the jacket put on. The joint portion is broken by adding a force on the tip portion of the ampoule with the jacket on from the lateral direction of the central axis or by twisting the tip portion. Then, the ampoule with a jacket on is attached to a needleless syringe. And the opening end is aimed closely at the skin where a liquid drug is administered.

Next, the movable stopper inside the ampoule is pushed with a plunger and the like. The drug solution pushed by the movable stopper is administered into a skin through an opening end.

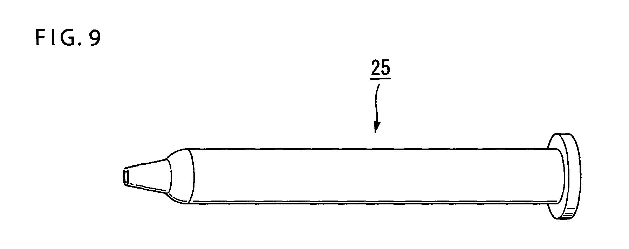

After the use of the needleless syringe, the ampoule (20) is taken out of the jacket (30), and the ampoule is discarded. Fig. 9 is a conceptual diagram showing an ampoule after use. As shown in the Fig. 9, since the ampoule (20) after use does not have an injection needle, infections caused by used injection needles can be prevented. It is also easily discarded because there is no need to separate an injection needle from the ampoule.

A known needleless syringe can be used as a needleless syringe. The specific examples of needleless syringes are as follows: a needleless syringe described in JP-A 2003-093508; a needleless syringe described in Japanese Patent No. 3574433; and a needleless syringe described in Japanese Patent No. 3560889. Japanese Patent No. 3574433 shows a needleless syringe for injecting active component comprising, from upstream to downstream: a propelling system which is composed of a shock wave generation apparatus; a barrier having an upstream surface and a down stream surface, and having at least a blind hole in the downstream surface wherefrom the active component can be input; a guide for applying the syringe to the skin of the patient, wherein the barrier, on one hand, is fixed and endures shock waves, and the barrier, on the other hand, propagates the shock waves properly. Japanese Patent No. 3560889 shows a needleless syringe for spraying drug solution by pushing a piston, which was added ahead power in a latch state in advance, into drug solution, which was taken in from an injection hole of a nozzle, in high speed. The syringe comprises: a front cylindrical portion incorporated with a nozzle having an injection hole; a concaved portion threadably mounted on the front cylindrical portion in the longitudinal direction; a rear cylindrical portion having the injection hole, and a concaved portion being provided in the rotational direction of the suction of the drug solution and being connected with the concaved portion at the other side of the end portion in the longitudal direction; a ball reception integrally provided on the rear cylindrical portion; multiple of balls in the ball reception; a piston driving rod on which latch channel is provided so that each part of the ball comes out and in, and the piston driving rod being retained in the rear cylindrical portion via the ball reception with the balls filled; a convex portion being inserted in the longitudinal concave portion or in the circumferential concave portion provided on the rear cylindrical portion, and a concave portion longitudinal to the rear cylindrical portion having a touching surface which is provided so that the surface touches the ball in a state that the ball is in the latch channel, and a release switch wherein the convex portion is movable along the circumferential concave portion; and a compression spring being provided between the rear cylindrical portion and the release switch. When the ball is not in the latch channel, the ball touches the front of the touching surface, and the release switch is held against the compression spring. When the ball is in the latch channel, the ball goes over the front of the touching surface, touches the touching surface, thereby holding the piston driving rod, and the convex portion of the release switch is drove toward the longitudinal direction to the end portion of the convex portion by the momentum of the compression spring. Then the ball reception and the ball roll, and the concave portion of the release switch goes into the circumferential convex portion via the touching surface, thereby preventing the pushing operation of the release switch.

### Example 1

Hereinafter, an example of actual production of a needleless syringe unit is explained. As the resin for producing an ampoule, the resin represented by the formula (I) was synthesized by varying the composition ratio between X and Y as appropriate. By so ding, the glass transition temperature was controlled. The one produced by the formula (I), wherein R¹ to R¹¹ were all hydrogen atoms was used. The synthetic raw material used was ethylene and bicycle [2.2.1] hept-2-en. Ampoules were produced by varying the ratio in various times. When the resin having the grass transition temperature ranging from 140°C to 180°C (more preferably from 145°C to 170°C, 150°C to 170°C, or 150°C to 160°C) was used, an ampoule having clarity and a preferred broken surface could be produced.

A mold comprises a pair of main molds and a sub mold. The main mold forms the external portion of the needleless ampoule, and the sub mold forms a through-hole such as a space portion.

Firstly, the main molds were separately set on the left side and the right side facing toward the molding, and were moved under a blow molding die. The die used is preferred to comprise a function of extruding parison, which is heated to the molding temperature, from an extrusion hole, and a function of injecting pressurized air such as compressed air from a blowing hole.

Next, parison, which was composed of hollow and half-dissolved thermoplastic material, was extruded between the main molds from the extrusion hole of the die. When the parison is extruded to a certain length, the main molds were joined. After the parison was cut by the bottom portion of the main molds, compressed air was inspired into the saclike parison, thereby blowing the parison to fit the inner surface of the main molds. In this way, an ampoule body of an ampoule usable as a needleless syringe can be formed. Next, the die was removed, and before the parison was cooled down, the sub mold was inserted in the center of a pouring portion and a grip portion, thereby forming a through-hole having a space portion. Finally, the main molds and the sub mold were removed from the parison, thereby forming an ampoule.

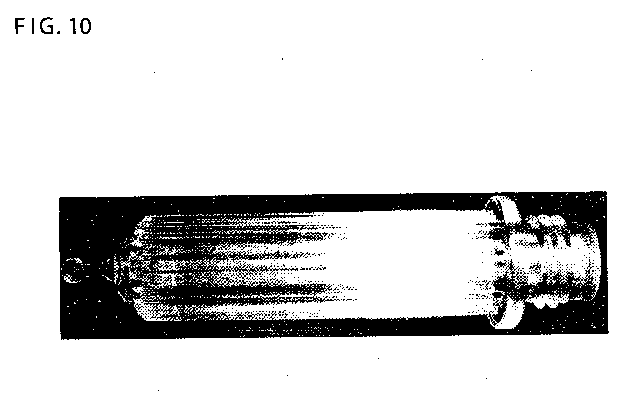

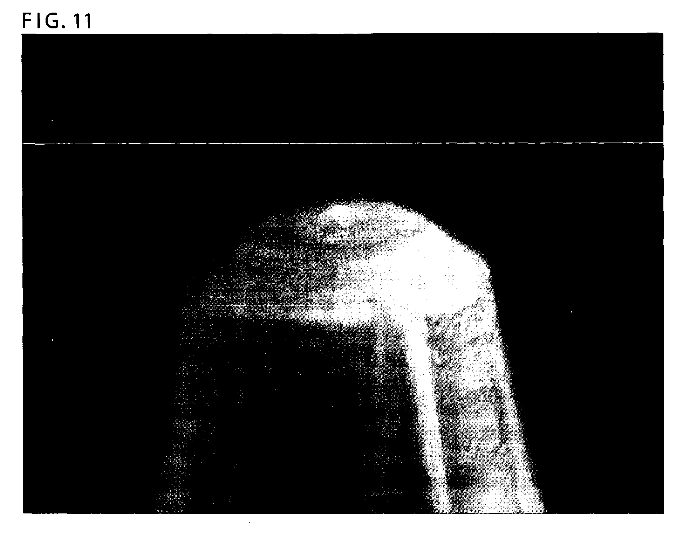

A jacket was produced in the same way as the ampoule. Fig. 10 is a photograph, replacing a diagram, showing a jacket and an ampoule comprising a needleless syringe unit. On the other hand, Fig. 11 is a photograph, replacing a diagram, showing an ampoule on which a jacket is put and then the joint portion of the ampoule was broken.

As above explained, since the end portion of a hermetically sealed container of the present invention is used as an end portion of a hermetically sealed container for a needleless syringe, it can preferably be used in the field of medical devices. In addition to be used as a needleless syringe in the field of medical devices, the end portion of a hermetically sealed container of the present invention can also be used as an end portion of a hermetically sealed container for preparing proper amount of medical agent for one prescription. It can also be used preferably in the field other than the medical devices.

## Claims

1. An end portion of a hermetically sealed container comprising:
a tip portion (1);
a truncated conical coupling portion (3) for coupling the tip portion (1) and a body portion (2); and
a joint portion (4) for coupling the tip portion (1) and the coupling portion (3), the joint portion (4) having narrower width than those of the tip portion (1) and the coupling portion (3),
wherein the tip portion (1), the joint portion (4), the coupling portion (3), and the body portion (2) are arranged coaxially to have a central axis (5),
wherein the tip portion (1), the joint portion (4), the coupling portion (3), and the body portion (2) have space portions (11, 14, 13, 12) being extended continuously from the body portion (2) to the middle of the tip portion (1), or space portions (14, 13, 12) being extended continuously from the body portion (2) to the middle of the joint portion (4),
wherein the diameter of the space portion at the joint portion (4) ranges from 5 µm to 1 mm, and
wherein the joint portion (4) is broken when a force is applied on the tip portion (1) from the lateral direction of the central axis (5), thereby exposing the head portion of the truncated conical coupling portion (3) and making the space portion (13) of the coupling portion a free end.

2. The end portion of a hermetically sealed container according to claim 1,
wherein the diameter of the space portion at the joint portion (4) ranges from 5 µm to 1x10⁻¹ mm, and
wherein the end portion of a hermetically sealed container is an end portion of an ampoule, the ampoule being used as a needleless syringe body.

3. The end portion of a hermetically sealed container according to claim 1,
wherein the tip portion (1) comprises a columnar body portion (1a) and a head portion (1b), the columnar body portion (1a) being joined with the joint portion (4), the head portion (1b) being provided at the tip of the body portion, the head portion (1b) having a spherical shape or having a columnar shape, the diameter of the columnar shape being larger than that of the columnar body portion (1a).

4. The end portion of a hermetically sealed container according to claim 1,
wherein the average outside diameter of the joint portion (4) ranges from 1/4 to 1/2 of the outside diameter of a head portion (3a) of the coupling portion (3).

5. The end portion of a hermetically sealed container according to claim 1,
wherein a head portion (3a) of the coupling portion has a flat portion perpendicularly intersecting with the central axis (5).

6. The end portion of a hermetically sealed container according to claim 1,
wherein the length of the joint portion (4) in the central axis (5) direction ranges from 1x10⁻³ mm to 1 mm.

7. The end portion of a hermetically sealed container according to claim 1,
wherein the tip portion (1), the joint portion (4), the coupling portion (3), and the body portion (2) are integrally formed,
wherein the tip portion (1), the joint portion (4), the coupling portion (3), and the body portion (2) are composed of resin composition represented by the below-described general formula (I), and the number average molecular weight of the resin composition ranges from 1x10³ to 1x10⁶: 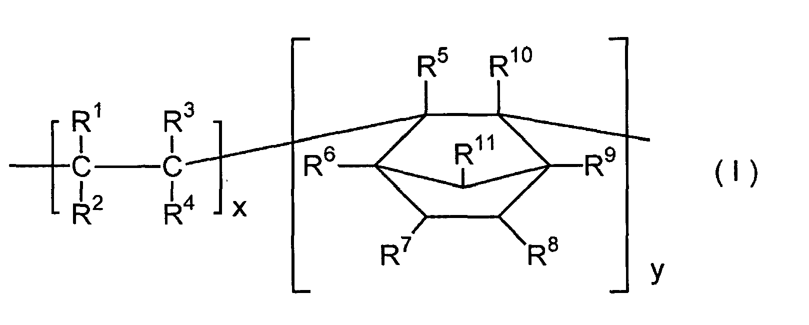 wherein R¹ to R⁴ are the same or different, and each represents a hydrogen atom, a C₁₋₃ alkyl group, a hydroxyl group, a methoxy group, an ethoxy group, a fluorine atom, a chlorine atom, or a bromine atom,
wherein R⁵ to R¹¹ are the same or different, and each represents a hydrogen atom, a C₁₋₃ alkyl group, a hydroxyl group, a methoxy group, an ethoxy group, a fluorine atom, a chlorine atom, or a bromine atom, or R⁷ and R⁸ together represents a C₄₋₆ bicyclo alkyl group, the C₄₋₆ bicyclo alkyl group has no substitution group or has one or more substitution groups selected from the group of "a methyl group, an ethyl group, a methoxy group, an ethoxy group, and a hydroxyl group", and
wherein x and y shows a ratio of substructure comprising ethylene based copolymer, and x:y is from 1:5 to 5:1.

8. The end portion of a hermetically sealed container according to claim 7,
wherein, in the formula (I), R¹ to R⁴ may be the same or different, and each represents a hydrogen atom, a methyl group, or a fluorine atom, and
wherein, in the formula (I), R⁵ to R¹¹ may be the same or different, and each represents a hydrogen atom, a methyl group, a hydroxyl group, a methoxy group, an ethoxy group, a fluorine atom, or a chlorine atom.

9. The end portion of a hermetically sealed container according to claim 7,
wherein the glass translation temperature of the resin composition ranges from 140°C to 180°C.

10. An ampoule (20) comprising:
the end portion (10) of a hermetically sealed container according to any one of claims 1 to 9; and
the body portion (2).

11. A ampoule (20) for a needleless syringe comprising:
the end portion (10) of a hermetically sealed container according to claim 1; and
the body portion (2).

12. A needleless syringe unit comprising:
the ampoule (20) according to claim 11; and
a jacket (30) in which the ampoule (20) can be inserted,
wherein a hole portion (31) is provided at the tip of the jacket (30), and
wherein the tip portion (1), the joint portion (4) and a head portion (3a) of the coupling portion, or the tip portion (1) and the joint portion (4) protrude from the hole portion (31), the hole portion (31) being provided at the tip of the jacket, when the ampoule (20) is inserted in the jacket (30).
